# EUROPEAN PATENT APPLICATION

(11) **EP 4 741 387 A1**
(43) Date of publication of application: **13.05.2026**
(21) Application number: 24835321.1
(22) Date of filing: 01.07.2024
(51) Int. Cl.: C07D 403/14, C07D 403/12, C07D 401/12, C07D 401/14, A61K 31/506, A61P 35/00

(54) **CRYSTAL FORM OF BIPYRIMIDINE COMPOUND, PREPARATION METHOD THEREFOR AND USE THEREOF**

(30) Priority: 05.07.2023 CN 202310819418
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: ZHOU, Lanping, Taizhou, Jiangsu 225316 (CN); GUO, Yushen, Taizhou, Jiangsu 225316 (CN); QIU, Jun, Taizhou, Jiangsu 225316 (CN); WU, Maojiang, Taizhou, Jiangsu 225316 (CN)
(74) Representative: Regimbeau
(86) International application number: PCT/CN2024/102920
(87) International publication number: WO 2025/007840

(57) **Abstract**

The present invention relates to a crystal form of a bipyrimidine compound and a preparation method therefor and a use thereof. In particular, the present invention relates to a crystal form I of 4'-cyclopropyl-5,6'-dimethoxy-N-((4-(1-methyl-4-(trifluoromethyl)-1H-imidazol-2-yl)bicyclic[2.2.2]oct-1-yl)methyl)-[2,5'-bipyrimidine]-4-amine, and the X-ray powder diffraction pattern of the crystal form I comprises characteristic peaks at diffraction angles (2θ) of 6.378±0.2°, 9.521±0.2°, 15.721±0.2°, 16.379±0.2°, 16.981±0.2°, 17.560±0.2°, 19.080±0.2°, and 22.200±0.2°. The crystal form I has high stability, high solubility, high plasma concentration, high bioavailability, and excellent pharmacological action, and is therefore suitable for pharmaceutical preparations.

## Description

### FIELD OF THE INVENTION

The present invention relates to a crystal form of bipyrimidine compound, a method for preparing the same and a use thereof.

### BACKGROUND OF THE INVENTION

Ubiquitination, as a dynamic and reversible process, involves a family of deubiquitinases (DUBs). There are approximately 100 DUBs in a human, which can be divided into a cysteine protease family and a metalloproteinase family. Among them, the cysteine protease family mainly includes ubiquitin-specific proteases (USPs), ubiquitin carboxy-terminal hydrolases (UCHs), Machado-Josephin domain proteases (MJDs), MINDY proteases (MINDYs), and ovarian tumor domain proteases (OTUs). The USPs family is the one representing the greatest quantity among known deubiquitinases, with over 50 types encoded by human genes. It plays a role in various physiological functions such as cell cycle, signal transduction, DNA damage repair, chromosome translocation, and gene transcription, by regulating protease substrates.

USP1 belongs to the USP subfamily of DUBs and does not exhibit significant activity on its own. However, it obtains complete enzymatic activity by binding to UAF1 to form a heterodimer complex (USP1/UAF1), and may regulate cellular targets in multiple pathways associated with cancer. For example, the USP1/UAF1 complex enables to deubiquitinate proliferating cell nuclear antigen (PCNA) mono-ubiquitinated by a key protein during translesion synthesis (TLS), to prevent excessive repair thereof, and enables to deubiquitinate fanconi anemia complementation group D2 (FANCD2) mono-ubiquitinated by a key protein in the fanconi anemia (FA) pathway, to prevent improper TLS repair in cells, thereby ensuring genomic stability, etc. These two DNA damage response (DDR) pathways are critical pathways for repairing DNA damage induced by a DNA crosslinker such as cisplatin, mitomycin, and ultraviolet radiation. USP1 can also interact with ID proteins and so on, so as to stabilize their expression in cells through deubiquitination. For example, in osteosarcoma cells, USP1 can deubiquitinate ID1, ID2, and ID3 to promote cell proliferation, and inhibition of USP1 can increase the sensitivity of osteosarcoma cells to chemotherapy. In addition, there are studies to demonstrate that USP1 is closely related to development of resistance to various drugs for tumor treatment. For example, in non-small cell lung cancer (NSCLC) cells with the resistance to cisplatin, USP1 has a high level of expression, and knock down of USP1 can significantly enhance cell sensitivity to cisplatin. In breast cancer cells, USP1 is highly expressed, promotes the proliferation of breast cancer cells and is closely related to the poor prognosis of breast cancer. As reported in the literature (J. Med. Chem. 2014, 57, 8099-8110, Synthesis and Structure-Activity Relationship Studies of N-Benzyl-2-phenylpyrimidin-4-amine Derivatives as Potent USP1/UAF1 Deubiquitinase Inhibitors with Anticancer Activity against Nonsmall Cell Lung Cancer), the compounds such as USP1 inhibitor ML323 can be used for non-small cell lung cancer. As reported in the literature (Cui S-Z, Lei Z-Y, Guan T-P, et al. Targeting USP1-dependent KDM4A protein stability as a potential prostate cancer therapy. Cancer Sci. 2020;00:1-15), USP1 inhibitors are used as potential therapeutic drugs for prostate cancer. In summary, USP1 is expected to become a hot target for treating various cancers and other diseases.

CN202310473956.2 discloses a series of USP1 inhibitors, particularly compound 104 having the following structure: 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine.

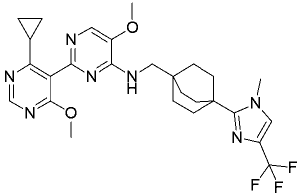

However, no reports on the crystal form of this compound have been found in the prior art.

### SUMMARY OF THE INVENTION

The technical problem to be solved by the present invention is to provide a crystal form of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine, a method for preparing the same and a use thereof. The inventors conducted extensive experimental research to discover a method capable of preparing a crystal form of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine, and X-ray powder diffraction analysis of the obtained crystal form revealed crystal form I with good stability.

The present invention solves the above technical problem through the following technical solutions:
The present invention provides crystal form I of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine, characterized in that the X-ray powder diffraction pattern thereof comprises characteristic peaks at diffraction angles (2θ) of 6.378±0.2°, 9.521±0.2°, 15.721±0.2°, 16.379±0.2°, 16.981±0.2°, 17.560±0.2°, 19.080±0.2° and 22.200±0.2°.

In some embodiments, the X-ray powder diffraction pattern of crystal form I further comprises at least one characteristic peak at diffraction angles (2θ) of 11.938±0.2°, 23.761±0.2°, 25.101±0.2° and 28.700±0.2°.

In some embodiments, the X-ray powder diffraction pattern of crystal form I comprises characteristic peaks at diffraction angles (2θ) of 6.378±0.2°, 9.521±0.2°, 11.938±0.2°, 15.721±0.2°, 16.379±0.2°, 16.981±0.2°, 17.560±0.2°, 19.080±0.2°, 22.200±0.2°, 23.761±0.2°, 25.101±0.2° and 28.700±0.2°.

In some embodiments, the X-ray powder diffraction pattern of crystal form I further comprises at least one characteristic peak at diffraction angles (2θ) of 10.239±0.2°, 12.400±0.2°, 14.882±0.2°, 20.420±0.2° and 25.980±0.2°.

In some embodiments, the X-ray powder diffraction pattern of crystal form I comprises characteristic peaks at diffraction angles (2θ) of 6.378±0.2°, 9.521±0.2°, 10.239±0.2°, 11.938±0.2°, 12.400±0.2°, 14.882±0.2°, 15.721±0.2°, 16.379±0.2°, 16.981±0.2°, 17.560±0.2°, 19.080±0.2°, 20.420±0.2°, 22.200±0.2°, 23.761±0.2°, 25.101±0.2°, 25.980±0.2° and 28.700±0.2°.

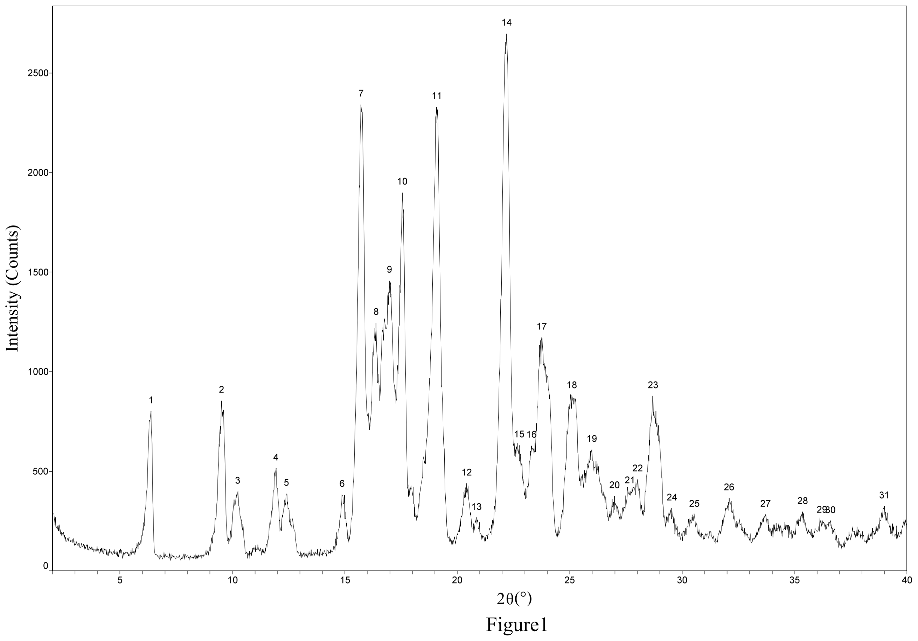

In some embodiments, the X-ray powder diffraction pattern of crystal form I is substantially as shown in Figure 1.

In some embodiments, the DSC spectrum of crystal form I shows one endothermic peak at 219.25±2°C.

In some embodiments, the TGA spectrum of crystal form I shows a weight loss of 0.10% from 20°C to 120°C, and a weight loss of 0.37% from 120°C to 250°C.

The present invention also provides a method for preparing crystal form I, comprising the following steps of:
mixing 4'-cyclopropyl-5,6'-dimethoxy-N-((4-(1-methyl-4-(trifluoromethyl)-1 H-imidazol-2-yl)bicyclo[2.2.2]octan-1-yl)methyl)-[2,5'-bipyrimidin]-4-amine and an organic solvent, and collecting a solid after solid-liquid separation of the resulting suspension;
the organic solvent is one or more selected from the group consisting of petroleum ether, C₄-C₆ ether and C₂-C₆ nitrile.

In some embodiments, the method comprises the following steps of:
triturating 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine in an organic solvent at 0 to 30°C for 10 minutes to 2 hours, and preferably at 25°C for 30 minutes, collecting a solid after solid-liquid separation of the resulting suspension, and optionally drying the solid;
the organic solvent is one or more selected from the group consisting of petroleum ether, methyl *tert-*butyl ether, tetrahydrofuran and acetonitrile, and preferably a mixture of petroleum ether and methyl *tert-*butyl ether;
advantageously, the ratio (w/v) of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine to the organic solvent is 1:5 to 1:1, and preferably 1:3.

The present invention also provides a method for preparing crystal form I, comprising the following steps of:
mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine and a solvent, and collecting a solid after solid-liquid separation of the resulting suspension.

In some embodiments, the method includes any one of the following methods:
method (I) comprises the following steps of:
   triturating 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine in a solvent, and collecting a solid after solid-liquid separation of the resulting suspension;
   the solvent is one or more selected from the group consisting of water, petroleum ether, C₁-C₆ alcohol, C₄-C₈ alkane, C₄-C₆ ether, C₃-C₆ ketone, C₃-C₈ ester, toluene and C₂-C₆ nitrile;
method (II) comprises the following steps of:
   mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine with a good solvent to dissolve, then mixing the resulting solution with an anti-solvent, and precipitating a crystal to obtain crystal form I;
   the good solvent is one or more selected from the group consisting of C₂-C₆ nitrile, methanol, benzyl alcohol, C₃-C₆ ketone, N-methylpyrrolidone, ethyl acetate, dichloromethane, N,N-dimethylformamide, dimethyl sulfoxide and tetrahydrofuran;
   the anti-solvent is one or more selected from the group consisting of petroleum ether, C₄-C₆ ether and C₂-C₆ alcohol;
method (III) comprises the following steps of:
   mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine and a solvent, cooling the resulting mixture, and precipitating a crystal to obtain crystal form I;
   the solvent is one or more selected from the group consisting of C₂-C₆ nitrile, C₁-C₃ alcohol, benzyl alcohol, C₃-C₆ ketone, N-methylpyrrolidone, C₃-C₈ ester, dichloromethane, N,N-dimethylformamide, dimethyl sulfoxide, tetrahydrofuran, methyltetrahydrofuran and 1,4-dioxane.

In some embodiments, the method (I) comprises the following steps of:
triturating 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine in a solvent at 0 to 50°C for 10 minutes to 7 days, and preferably at 25 to 30°C for 30 minutes to 4 days, collecting a solid after solid-liquid separation of the resulting suspension, and optionally drying the solid;
the solvent is one or more selected from the group consisting of water, methanol, n-heptane, ethyl acetate, ethylene glycol, isopropyl acetate, methyl isobutyl ketone, toluene, petroleum ether, methyl *tert-*butyl ether, tetrahydrofuran and acetonitrile,
preferably, the solvent is a mixture of petroleum ether and methyl *tert-*butyl ether, a mixture of water and ethanol, a mixture of *n*-heptane and ethyl acetate, a mixture of ethylene glycol and methanol, isopropyl acetate, methyl isobutyl ketone and toluene,
more preferably, the volume ratio of petroleum ether to methyl *tert-*butyl ether is 1:10 to 10:1, and preferably 1:1 to 10:1; the volume ratio of water to ethanol is 5:1 to 30:1, and preferably 10:1 to 20:1; the volume ratio of *n*-heptane to ethyl acetate is 5:1 to 30:1, and preferably 10:1 to 20:1; and the volume ratio of ethylene glycol to methanol is 5:1 to 30:1, and preferably 10:1 to 20:1,
advantageously, the ratio (w/v) of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine to the solvent is 1 g:1 mL to 1 g:20 mL, and preferably 1 g:3 mL to 1 g:10 mL.

In some embodiments, the method (II) comprises the following steps of:
mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine with a good solvent at 5 to 45°C, preferably at room temperature to dissolve, then mixing the resulting solution with an anti-solvent, and precipitating a crystal to obtain crystal form I;
the good solvent is one or more selected from the group consisting of methanol, acetone, ethyl acetate, dichloromethane, N,N-dimethylformamide, N-methylpyrrolidone, dimethyl sulfoxide, tetrahydrofuran, acetonitrile and benzyl alcohol;
the anti-solvent is one or more selected from the group consisting of methyl *tert-*butyl ether, isopropanol, 1-butanol and ethylene glycol;
advantageously, the volume of the anti-solvent is 2 to 20 times, and preferably 3 to 10 times the volume of the good solvent;
more advantageously, the ratio (w/v) of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine to the anti-solvent is 50 mg:1 mL to 1 mg:1 mL, and preferably 50 mg:1 mL to 5 mg:1 mL.

In some embodiments, the method (II) comprises the following steps of:
mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine with a good solvent at 5 to 45°C, preferably at room temperature to obtain a saturated or near-saturated solution, optionally filtering the solution, then mixing the solution with an anti-solvent, preferably under stirring, and precipitating a crystal to obtain crystal form I.

In some embodiments, the method (III) comprises the following steps of:
mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine and a solvent, cooling the resulting mixture, and precipitating a crystal to obtain crystal form I;
the solvent is a good solvent, and preferably the solvent is one or more selected from the group consisting of ethanol, methyl isobutyl ketone, isopropyl acetate, methyltetrahydrofuran and 1,4-dioxane.

Preferably, the method (III) comprises the following steps of:
mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine with a good solvent to dissolve, optionally filtering the resulting mixture, first cooling the filtrate until a solid precipitates, then further cooling the filtrate, and precipitating a crystal to obtain crystal form I.

Preferably, the method (III) comprises the following steps of:
mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine with a good solvent at 25 to 60°C, preferably at 30 to 50°C to dissolve, optionally filtering the resulting mixture, first cooling the filtrate to 5 to 35°C, preferably to room temperature, then placing it at -5 to 5°C, preferably at 4°C, and precipitating a crystal to obtain crystal form I;
more preferably, the ratio (w/v) of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine to the solvent is 100 mg:1 mL to 10 mg:1 mL, and preferably 80 mg:1 mL to 30 mg:1 mL.

The present invention also provides a pharmaceutical composition comprising crystal form I and one or more pharmaceutically acceptable carrier(s).

The present invention relates to a use of crystal form I or the pharmaceutical composition comprising the same in the preparation of a medicament for treating or preventing diseases or conditions associated with inhibition of ubiquitin-specific protease 1 (USP1).

The present invention also relates to a use of crystal form I or the pharmaceutical composition comprising the same in the preparation of a medicament for treating or preventing cancer.

The present invention also relates to crystal form I or the pharmaceutical composition comprising the same, for use in treating or preventing diseases or conditions associated with inhibition of ubiquitin-specific protease 1 (USP1).

The present invention also relates to crystal form I or the pharmaceutical composition comprising the same, for use in treating or preventing cancer.

The present invention also relates to a method for treating or preventing diseases or conditions associated with inhibition of ubiquitin-specific protease 1 (USP1), comprising a step of administering an effective amount of crystal form I or the pharmaceutical composition comprising the same to a patient in need thereof.

The present invention also relates to a method for treating or preventing cancer, comprising a step of administering an effective amount of crystal form I or the pharmaceutical composition comprising the same to a patient in need thereof.

In some embodiments, the cancer is selected from the group consisting of lung cancer, non-small cell lung cancer (NSCLC), colon cancer, bladder cancer, osteosarcoma, ovarian cancer, skin cancer, and breast cancer.

The expression "pharmaceutically acceptable" as described in the present disclosure is one which is useful for preparing a pharmaceutical composition that is generally safe, have neither biological toxicity nor other undesirable toxicity, and are acceptable for veterinary use and human pharmaceutical use.

The term "carrier" as described in the present disclosure refers to a diluent, adjuvant or excipient administered together with the compound, including but not limited to a filler, disintegrant, lubricant, suspending agent, binder, sweetener, flavoring agent, preservative, matrix, and the like. The composition of the present invention can be prepared by any method known in the art, so as to provide rapid, sustained or slow release of the active ingredient after administration to a patient.

### DESCRIPTION OF THE DRAWINGS

Figure 1 is the X-ray powder diffraction (XRPD) pattern of crystal form I.
Figure 2 is the differential scanning calorimetry (DSC) spectrum of crystal form I.
Figure 3 is the thermogravimetric analysis (TGA) spectrum of crystal form I.
Figure 4 is the PLM spectrum of crystal form I (10X10X).
Figure 5 is the PLM spectrum of crystal form I (10X40X).
Figure 6 is the DVS result of crystal form I.
Figure 7 is the XRPD patterns of crystal form I before and after DVS. In this figure, from top to bottom, they are the XRPD pattern after DVS and the XRPD pattern before DVS.

### DETAILED DESCRIPTION OF THE INVENTION

The present invention will be set out in more detail through examples below. The examples of the present invention are merely intended to describe the technical solutions of the present invention, and should not be considered as limiting the spirit and scope of the present invention.

The purity analysis method is as follows: a Kinetex EVO C18 (50×4.6 mm, 5 µm, 100 Å) chromatographic column is used, acetonitrile-water is used as the mobile phase for gradient elution, the flow rate is 1.5 mL/min, and the detection wavelength is 220 nm.

MS is determined by a LC (Agilent 1260 Infinity II)/MS (G6125B single quadrupole) mass spectrograph (manufacturer: Agilent) (Photodiode Array Detector).

The structures of the compounds are identified by hydrogen nuclear magnetic resonance, and the equipment model is WNMR-I-400 MHz.

Preparative liquid chromatography is conducted on an Agilent 1260 Infinity II high performance liquid chromatograph (manufacturer: Agilent). The chromatographic column is Daisogel C18 10 µm 100A (30 mm×250 mm), and the mobile phase is acetonitrile/water.

GF254 silica gel plate of Qingdao Haiyang Chemical is used for the thin-layer silica gel chromatography (TLC). The dimension of the silica gel plate used in TLC is 0.20 mm to 0.25 mm, and the dimension of the silica gel plate used in product purification is 0.5 mm.

Silica gel of 100 to 200 mesh, 200 to 300 mesh or 300 to 400 mesh of Qingdao Haiyang Chemical is used as a carrier for gel column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Shanghai Innochem Science & Technology, Energy Chemical, Shanghai Bide Pharmatech, and the like.

Unless otherwise specified in the examples, the reactions are carried out under nitrogen atmosphere.

Nitrogen atmosphere means that a reaction flask is equipped with a nitrogen balloon (about 1 L).

Unless otherwise specified in the examples, a solution means an aqueous solution.

The chemical reactions described in the present invention are generally carried out under normal pressure. Unless otherwise specified in the examples, the reaction temperature is room temperature, that is, 20°C to 30°C.

Unless otherwise defined, all professional and scientific terms used herein have the same meaning as those familiar to those skilled in the art. In addition, any methods and materials similar or equivalent to the content described herein can be applied to the method of the present invention.

Unless otherwise specified, the mixing ratios of different solvents are volume ratios.

### X-ray powder diffraction (XRPD)

The instrument is Shimadzu XRD-6000, and the samples are scanned according to the following parameters:
The ray source is Cu~Kα target (1.54056 Å).

The minimum operating voltage and current of light tube are 40 kV and 30 mA, respectively.

The 2-theta value of the sample scanning range is 2° to 40°. The scanning speed is 4°/min.

### Thermogravimetric analysis (TGA)

The instrument is PerkinElmer Pyrisl TGA. The analysis is carried out at 20°C to 350°C with a heating rate of 10°C/min, and is kept at 350°C for 1 minute.

### Differential scanning calorimetry (DSC)

The instrument is METTLER DSC3. Differential scanning calorimetry is carried out at 30°C to 300°C, and is kept at 300°C for 1 minute. The heating rate is 10°C/min.

### Polarized light microscope (PLM)

The PLM instrument is XPV-203E from Shanghai Changfang Optical Instrument Co., Ltd.

The sample is observed using a 10X eyepiece and a 10X or 40X objective lens. Images are recorded using a camera computer system.

### Dynamic vapor sorption (DVS)

The DVS instrument is SMS DVS Intrinsic.

The moisture absorption/desorption characteristics are tested in an aqueous environment at 25°C under a cyclic relative humidity (RH) of 0%~90%~0%.

### HPLC

The HPLC for solubility and stability is carried out using a Phenomenex Luna C18 (2), 3 µm, 2X50 mm chromatographic column.

### Example 1: Synthesis of 4'-cyclopropyl-5,6'-dimethoxy-N-((4-(1-methyl-4-(trifluoromethyl)-1H-imidazol-2-yl)bicyclo [2.2.2] octan-1-yl)methyl)-[2,5'-bipyrimidin]-4-amine

### 1.1 Synthesis of General Intermediate A2-7 ((4-cyclopropyl-6-methoxypyrimidin-5-yl)boronic acid)

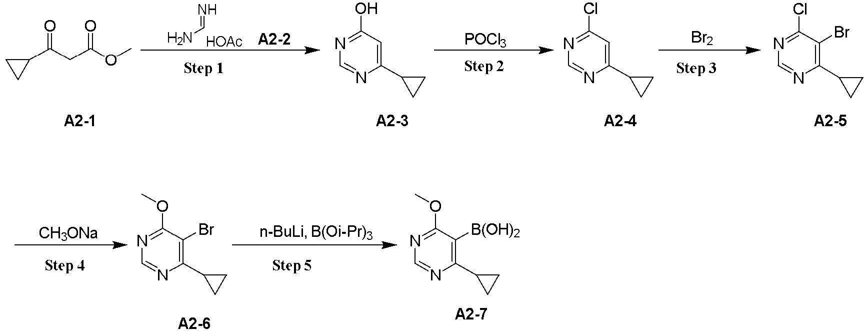

### Step 1: Synthesis of Compound A2-3 (6-cyclopropylpyrimidin-4-ol)

Compound **A2-1** (200.0 g, 1.4 mol, 1.00 *eq*) and compound **A2-2** (292.0 g, 2.81 mol, 2.00 *eq*) were added to methanol (1.2 L), and a solution of sodium methoxide in methanol (5.4 M, 1.3 L, 5.00 *eq*) was added at 0°C. After the addition was completed, the reaction was heated up to 20°C and stirred. Glacial acetic acid was added at 0°C to adjust the pH to 7-8. The resulting mixture was concentrated under vacuum to obtain a crude product, which was purified by column chromatography (eluent: petroleum ether/ethyl acetate = 1/0 - 1/1) to obtain compound **A2-3** (90.3 g). LC-MS: m/z = 137.1 (M+H)⁺.

### Step 2: Synthesis of Compound A2-4 (4-chloro-6-cyclopropylpyrimidine)

Compound **A2-3** (40.0 g, 293.0 mmol, 1.00 *eq)* was added to phosphorus oxychloride (180.0 mL), and the reaction mixture was heated up to 60°C and stirred. The reaction solution was concentrated under vacuum to obtain a crude product. The crude product was dissolved in ethyl acetate (400.0 mL) and water (400.0 mL), and extracted with ethyl acetate (400.0 mL). The organic layer was dried, and filtered to obtain a filtrate. The filtrate was concentrated under vacuum to obtain a crude product, which was purified by column chromatography (eluent: petroleum ether/ethyl acetate = 20/1 - 1/1) to obtain compound **A2-4** (11.0 g). LC-MS: m/z = 155.0 (M+H)⁺.

### Step 3: Synthesis of Compound A2-5 (5-bromo-4-chloro-6-cyclopropylpyrimidine)

Compound **A2-4** (11.0 g, 71.1 mmol, 1.00 *eq*) was dissolved in methanol (150.0 mL), and bromine (34.1 g, 213.0 mmol, 3.00 *eq*) was added at -60°C. The reaction was heated up to 20°C and stirred. Then, saturated sodium bicarbonate solution (200.0 mL) and water (100.0 mL) were added at 0°C, the resulting mixture was extracted with dichloromethane (200.0 mL) three times. The organic layer was washed with water, dried, and filtered to obtain a filtrate. The filtrate was concentrated under vacuum to obtain a crude product, which was purified by column chromatography (eluent: petroleum ether/ethyl acetate = 1/0 - 3/1) to obtain compound **A2-5** (7.8 g). ¹H NMR (400 MHz, CDCl₃) δ 8.61 (s, 1H), 2.56 - 2.62 (m, 1H), 1.23 - 1.26 (m, 2H), 1.16 - 1.21 (m, 2H); LC-MS: m/z = 232.9 (M+H)⁺.

### Step 4: Synthesis of Compound A2-6 (5-bromo-4-cyclopropyl-6-methoxypyrimidine)

Compound **A2-5** (7.8 g, 33.4 mmol, 1.00 *eq*) was dissolved in methanol (240.0 mL), and sodium methoxide (18.0 g, 100.0 mmol, 3.00 *eq*) was added at 0°C. The reaction was heated up to 30°C and stirred. The reaction solution was concentrated under vacuum to obtain a crude product, which was purified by column chromatography (eluent: petroleum ether/ethyl acetate = 10/1 - 5/1) to obtain compound **A2-6** (7.3 g). LC-MS: m/z = 228.9 (M+H)⁺.

### Step 5: Synthesis of Compound A2-7 ((4-cyclopropyl-6-methoxypyrimidin-5-yl)boronic acid)

Compound **A2-6** (10.3 g, 44.9 mmol, 1.00 *eq*) and triisopropyl borate (11.8 g, 62.9 mmol, 1.40 *eq*) were dissolved in tetrahydrofuran (30.0 mL) and toluene (90.0 mL), and *n*-butyl lithium (2.5 M, 25.1 mL, 1.40 *eq*) was added dropwise at -70°C. The reaction was stirred at -70°C. Then, 1 N hydrochloric acid solution (50.0 mL) was added dropwise at -70°C. The reaction was heated up to 20°C and stirred. Saturated aqueous sodium bicarbonate solution was then added to adjust the pH to 7-8, and the resulting mixture was extracted with ethyl acetate (100.0 mL) three times. The organic layer was washed with water, dried, and filtered to obtain a filtrate, which was concentrated under vacuum to obtain compound **A2-7** (6.9 g). ¹H NMR (400 MHz, CDCl₃) δ 8.52 (s, 1H), 8.45 (s, 2H), 3.84 (s, 3H), 1.88 - 1.92 (m, 2H), 0.93 - 1.01 (m, 4H).

### 1.2 General Intermediate BB2 (2-chloro-5-methoxypyrimidin-4-amine)

Intermediate **BB2** was purchased from Leyan Reagents.

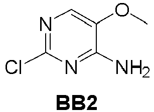

### 1.3 Synthesis of General Intermediate C35 ((4-(1-methyl-4-(trifluoromethyl)-1H-imidazol-2-yl)bicyclo[2.2.2]octan-1-yl)methyl-4-methylbenzenesulfonate)

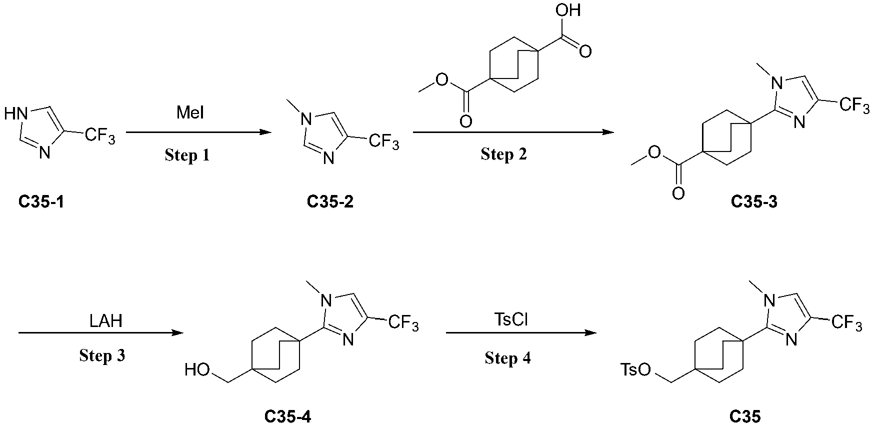

### Step 1: Synthesis of Compound C35-2 (1-methyl-4-(trifluoromethyl)-1H-imidazole)

Compound 4-(trifluoromethyl)-1H-imidazole (compound **C35-1,** 26 g, 0.19 mol) and potassium carbonate (105.5 g, 0.76 mol, 4.0 eq) were added to acetonitrile (390 mL). Iodomethane (32.6 g, 0.23 mol, 1.2 eq) was added dropwise at 0°C, and the reaction mixture was stirred at room temperature. Then, the reaction solution was concentrated under vacuum to obtain a crude product, which was extracted three times with ethyl acetate (200 mL). The organic layer was washed with water, dried, and filtered to obtain a filtrate, which was concentrated under vacuum to obtain compound **C35-2** (26 g, crude). LC-MS: m/z = 151.0 (M+H)⁺.

### Step 2: Synthesis of Compound C35-3 (methyl 4-(1-methyl-4-(trifluoromethyl)-1H-imidazol-2-yl)bicyclo[2.2.2]octane-1-carboxylate)

Compound **C35-2** (14.75 g, 98.3 mmol) and monomethyl hydrogen bicyclo[2.2.2]octane-1,4-dicarboxylate (25 g, 117.9 mmol, 1.2 eq) were dissolved in dichloromethane (295 mL) and water (295 mL), followed by the addition of silver nitrate (6.0 g, 35.4 mmol, 0.3 eq.) and ammonium persulfate (44.9 g, 78.7 mmol, 2.0 eq.). The reaction mixture was stirred at 25°C, filtered through diatomaceous earth, and extracted three times with dichloromethane (200 mL). The organic layer was washed with water, dried, and filtered to obtain a filtrate, which was concentrated under vacuum to obtain compound **C35-3** (35.2 g, crude) as a yellow oil. ¹H NMR (400MHz, CDCl₃) 7.09 - 7.08 (m, 1H), 3.77 (s, 3H), 3.67 (s, 3H), 2.07 - 2.03 (m, 6H), 1.93 - 1.89 (m, 6H); LC-MS: m/z = 317.2 (M+H)⁺.

### Step 3: Synthesis of Compound C35-4 ((4-(1-methyl-4-(trifluoromethyl)-1H-imidazol-2-yl)bicyclo[2.2.2]octan-1-yl)methanol)

Compound **C35-3** (35.2 g, 111.4 mmol) was dissolved in tetrahydrofuran (180.0 mL), and lithium aluminum hydride (2.5 M, 111.4 mL, 278.5 mmol, 2.5 eq) was added at 0°C. The reaction mixture was heated up to 20°C and stirred. Then, ice water (50.0 mL) was added at 0°C, the resulting mixture was extracted three times with ethyl acetate (500.0 mL). The organic layer was dried, and filtered to obtain a filtrate, which was concentrated under vacuum to obtain compound **C35-4** (25.1 g, crude) as a yellow oil. LC-MS: m/z = 289.1 (M+H)⁺.

### Step 4: Synthesis of Compound C35

Compound **C35-4** (25.1 g, 87.2 mmol), *p*-toluenesulfonyl chloride (33.2 g, 174.4 mmol, 2.0 eq) and 4-dimethylaminopyridine (32.0 g, 261.6 mmol, 3.0 eq) were added to dichloromethane (251 mL) successively, and the reaction mixture was stirred at 25°C. The reaction solution was washed with water, dried, and filtered to obtain a filtrate. The filtrate was concentrated under vacuum to obtain a crude product, which was purified by column chromatography (eluent: petroleum ether/dichloromethane = 1/0 - 0/1) to obtain intermediate **C35** (10.0 g) as a white solid. ¹H NMR (400 MHz, DMSO-d6) δ 7.82 (d, J = 8.2 Hz, 2H), 7.67 (s, 1H), 7.52 (d, J = 8.0 Hz, 2H), 3.77 (s, 3H), 3.72 (s, 2H), 2.46 (s, 3H), 1.95 - 1.86 (m, 6H), 1.49 - 1.37 (m, 6H); LC-MS: m/z = 443.3 (M+H)⁺.

### 1.4 Synthesis of Intermediate BB2C35 (2-chloro-5-methoxy-N-((4-(1-methyl-4-(trifluoromethyl)-1H-imidazol-2-yl)bicyclo [2.2.2] octan-1-yl)methyl)pyrimidin-4-amine)

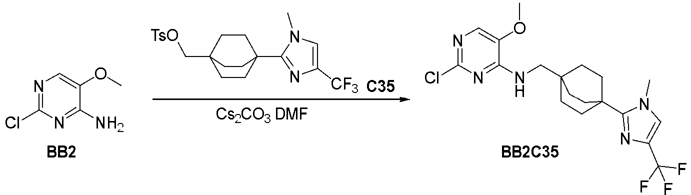

Compound **BB2** (1.0 g, 6.29 mmol, 1.00 eq), compound **C35** (2.78 g, 6.29 mmol, 1.0 eq) and cesium carbonate (10.2 g, 31.45 mmol, 5.0 eq) were added to N,N-dimethylformamide (20.0 mL) successively, and the reaction mixture was stirred at 130°C. The reaction solution was cooled to 25°C. Dichloromethane (100.0 mL) was added, and the reaction solution was filtered to obtain a filtrate. The filtrate was washed with water, dried, and concentrated under vacuum to obtain a crude product, which was purified by column chromatography (eluent: petroleum ether/dichloromethane = 1/0 - 0/1) to obtain intermediate **BB2C35** (1.03 g, 2.41 mmol, 38.3% yield) as a white solid. LC-MS: m/z = 430.1 (M+H)⁺.

### 1.5 Synthesis of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine

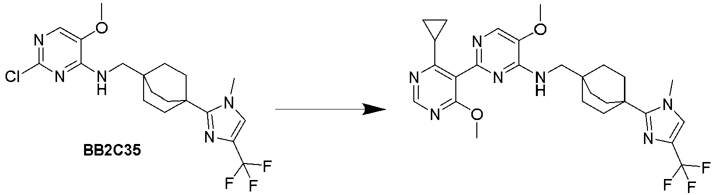

Compound BB2C35 (85.8 g, 0.2 mol), compound A2-7 (48.5 g, 0.25 mol, 1.25 eq.), ligand XPhos (37.4 g, 0.08 mol, 0.4 eq.), potassium phosphate (169.8 g, 0.8 mol, 4 eq.), and catalyst XPhos-Pd-G2 (31.5 g, 0.04 mol, 0.2 eq.) were added to 1,4-dioxane (1026 mL)/water (171.6 mL) successively. The reaction mixture was heated up to 85°C and stirred under nitrogen atmosphere. The mixture was concentrated under reduced pressure, cooled to 25°C, and dissolved in water. Then, the resulting solution was extracted with ethyl acetate (1026 mL), dried, and concentrated under reduced pressure to obtain about 200 g of the crude title compound.

Wherein: XPhos is 2-dicyclohexylphosphino-2',4',6'-triisopropylbiphenyl; XPhos-Pd-G2 is chloro(2-dicyclohexylphosphino-2',4',6'-triisopropyl-1,1'-biphenyl)[2-(2'-amino-1,1'-biphenyl)]palladium(II).

### Example 2: Preparation of Crystal Form I of 4'-cyclopropyl-5,6'-dimethoxy-N-((4-(1-methyl-4-(trifluoromethyl)-1H-imidazol-2-yl)bicyclo [2.2.2] octan-1-yl)methyl)-[2,5'-bipyrimidin]-4-amine

Approximately 200 g of the crude product 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine obtained in Example 1 was dissolved in 1 L (5V) of ethyl acetate. Silica gel was then added, and the resulting mixture was concentrated under vacuum until it became a powder. The powder was purified by silica gel column chromatography (eluent: dichloromethane: methanol = 100:5) to obtain approximately 91 g of a pale yellow solid. The solid was then triturated in 275 ml of solvent (petroleum ether: methyl *tert-*butyl ether = 10:1) at 25°C for 30 minutes. The resulting mixture was filtered, and dried under vacuum at 50°C to obtain 85.0 g of a white powder solid, with a yield of 78% and a purity of 99.2%.
LC-MS: m/z = 544.3 (M+H)⁺;
¹H NMR (400 MHz, DMSO-*d*₆) δ 8.62 (s, 1H), 7.90 (s, 1H), 7.65 (d, *J =* 1.5 Hz, 1H), 6.99 (t, *J =* 6.5 Hz, 1H), 3.93 (s, 3H), 3.84 (s, 3H), 3.77 (s, 3H), 3.21 (d, *J =* 6.5 Hz, 2H), 1.90 (m, 6H), 1.74 (m, 1H), 1.48 (m, 6H), 1.06 - 1.01 (m, 2H), 0.92 - 0.85 (m, 2H).

After X-ray powder diffraction analysis, the crystal form was defined as crystal form I. The X-ray powder diffraction data are shown in Table 1, and the X-ray powder diffraction pattern is shown in Figure 1.

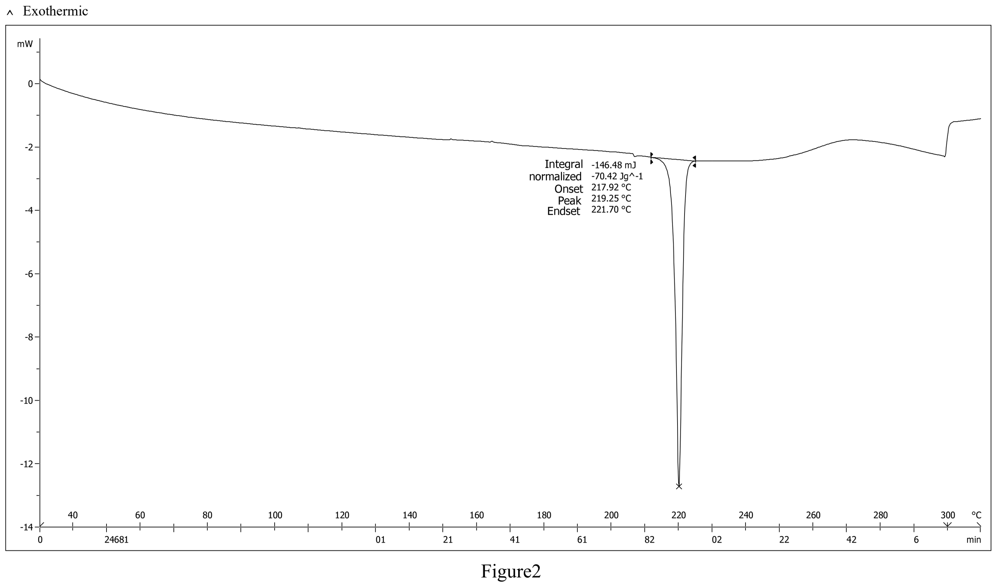

The DSC spectrum of crystal form I is shown in Figure 2, which shows a distinct endothermic peak at 219.25±2°C.

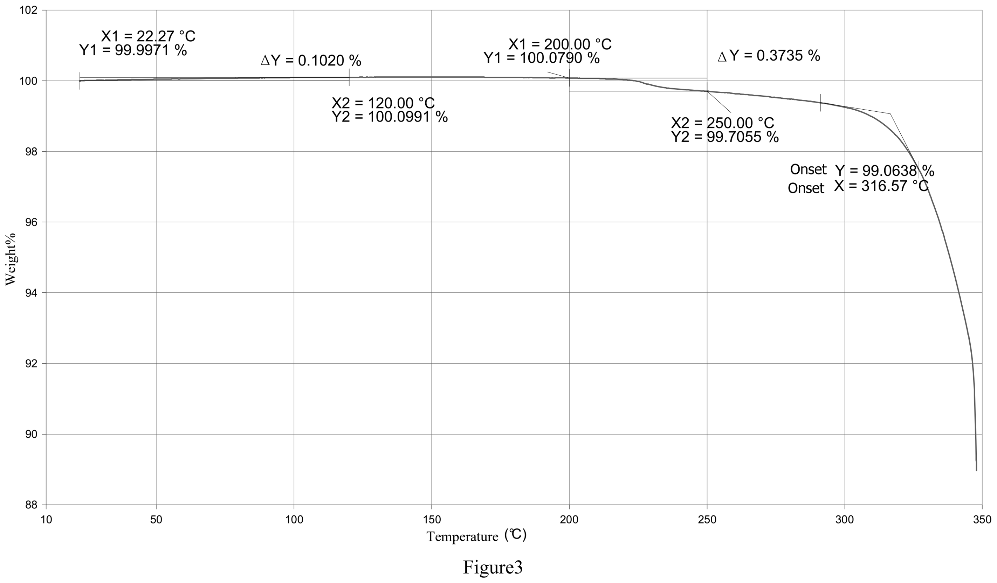

The TGA spectrum of crystal form I is shown in Figure 3, which shows a weight loss of 0.10% from 20°C to 120°C, and a weight loss of 0.37% from 120°C to 250°C.

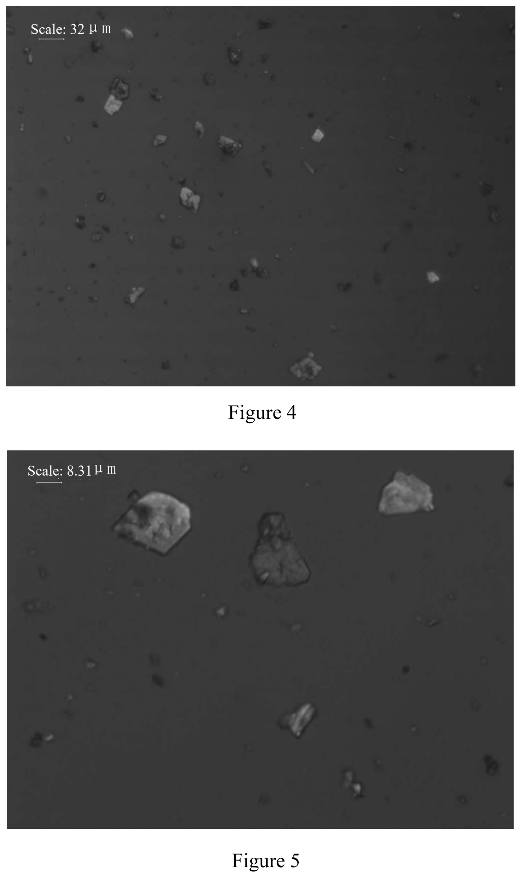

The PLM result of crystal form I is shown in Figures 4 and 5.

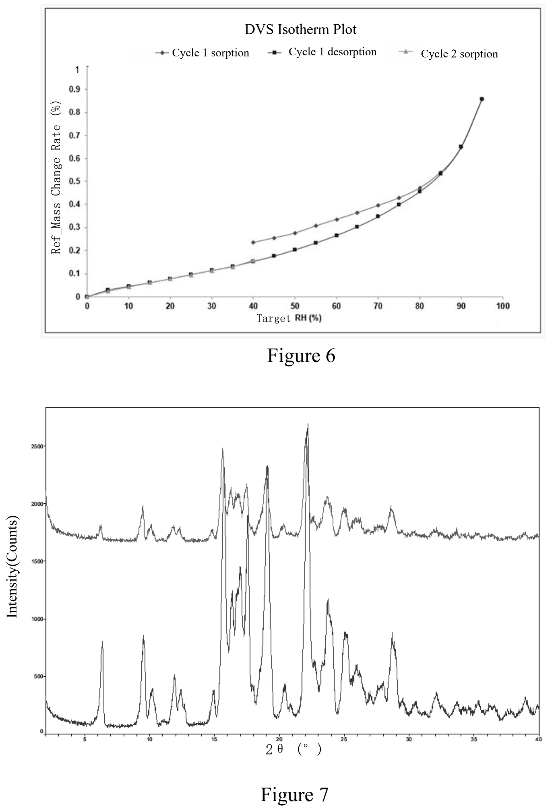

The DVS result of crystal form I is shown in Figure 6, which shows a water absorption rate of 0.02% from 0% RH to 95% RH, indicating that crystal form I is non-hygroscopic. The XRPD pattern of the crystal form after DVS is shown in Figure 7, which shows that the crystal form did not change after DVS.

**Table 1**

| Peak No. | 2θ | d value | Peak height | Peak height % | Area | Area % |
|---|---|---|---|---|---|---|
| 1 | 6.378 | 13.8461 | 727 | 28.6 | 10849 | 16.5 |
| 2 | 9.521 | 9.2814 | 781 | 30.7 | 14097 | 21.4 |
| 3 | 10.239 | 8.6325 | 280 | 11.0 | 5181 | 7.9 |
| 4 | 11.938 | 7.4071 | 422 | 16.6 | 8586 | 13.0 |
| 5 | 12.400 | 7.1320 | 297 | 11.7 | 7386 | 11.2 |
| 6 | 14.882 | 5.9479 | 235 | 9.3 | 2901 | 4.4 |
| 7 | 15.721 | 5.6325 | 2179 | 85.8 | 42619 | 64.8 |
| 8 | 16.379 | 5.4075 | 862 | 33.9 | 29552 | 44.9 |
| 9 | 16.981 | 5.2170 | 885 | 34.8 | 30384 | 46.2 |
| 10 | 17.560 | 5.0463 | 1631 | 64.2 | 27167 | 41.3 |
| 11 | 19.080 | 4.6476 | 2134 | 84.0 | 51778 | 78.7 |
| 12 | 20.420 | 4.3456 | 287 | 11.3 | 6517 | 9.9 |
| 13 | 20.860 | 4.2550 | 103 | 4.1 | 1535 | 2.3 |
| 14 | 22.200 | 4.0010 | 2540 | 100.0 | 65794 | 100.0 |
| 15 | 22.759 | 3.9040 | 232 | 9.1 | 6731 | 10.2 |
| 16 | 23.301 | 3.8144 | 267 | 10.5 | 12603 | 19.2 |
| 17 | 23.761 | 3.7416 | 987 | 38.9 | 36476 | 55.4 |
| 18 | 25.101 | 3.5448 | 693 | 27.3 | 22011 | 33.5 |
| 19 | 25.980 | 3.4267 | 206 | 8.1 | 5352 | 8.1 |
| 20 | 27.000 | 3.2996 | 104 | 4.1 | 804 | 1.2 |
| 21 | 27.680 | 3.2201 | 111 | 4.4 | 4070 | 6.2 |
| 22 | 28.018 | 3.1820 | 169 | 6.7 | 4128 | 6.3 |
| 23 | 28.700 | 3.1080 | 603 | 23.7 | 15095 | 22.9 |
| 24 | 29.540 | 3.0214 | 87 | 3.4 | 513 | 0.8 |
| 25 | 30.539 | 2.9249 | 112 | 4.4 | 2525 | 3.8 |
| 26 | 32.100 | 2.7861 | 215 | 8.5 | 6467 | 9.8 |
| 27 | 33.718 | 2.6560 | 108 | 4.3 | 2547 | 3.9 |
| 28 | 35.360 | 2.5363 | 114 | 4.5 | 1797 | 2.7 |
| 29 | 36.221 | 2.4780 | 89 | 3.5 | 3082 | 4.7 |
| 30 | 36.599 | 2.4533 | 102 | 4.0 | 3070 | 4.7 |
| 31 | 38.998 | 2.3077 | 154 | 6.1 | 3381 | 5.1 |

### Examples 3 to 10: Preparation of Crystal Form I (Anti-solvent Method)

Approximately 50 mg of crystal form I was weighed, and dissolved at room temperature in an appropriate volume of the good solvent shown in Table 2 to obtain a saturated or near-saturated solution. The solution was filtered, and the anti-solvent shown in Table 2 was slowly added under magnetic stirring with a volume of 3 to 10 times the volume of the good solvent. The resulting mixture was stirred until a sufficient amount of solid precipitated, and then centrifuged to collect the solid. The resulting solid was dried under reduced pressure (40°C).

**Table 2**

| **Good solvent / volume (mL)** | **Anti-solvent / volume (mL)** |
|---|---|
| Methanol /1.2 | Methyl *tert-*butyl ether /9 |
| Acetone /0.8 | Methyl *tert-*butyl ether /6 |
| Ethyl acetate /2.0 | Isopropanol /10 |
| Dichloromethane /0.2 | 1-Butanol /3 |
| N,N-Dimethylformamide /0.3 | Isopropanol /4 |
| N-Methylpyrrolidone /0.3 | 1-Butanol /4 |
| Dimethyl sulfoxide /1.0 | Isopropanol /8 |
| Tetrahydrofuran /0.4 | Methyl *tert-*butyl ether /4 |
| Acetonitrile /2.4 | Ethylene glycol /12 |
| Benzyl alcohol /0.3 | Methyl *tert-*butyl ether /3 |

X-ray powder diffraction analysis showed that the crystal forms obtained in Examples 3 to 10 were all crystal form I.

### Examples 11 to 16: Preparation of Crystal Form I (Suspension-trituration Method)

Approximately 50 mg of crystal form I was weighed, and an appropriate volume of the solvent shown in Table 3 was added. The resulting mixture was stirred magnetically (400 rpm) at 50°C for 4 days, and then centrifuged to collect the solid.

**Table 3**

| **Solvent** | **Volume (mL)** |
|---|---|
| Water/methanol (v:v=95:5) | 0.5 |
| *n*-Heptane/Ethyl Acetate (v:v=95:5) | 0.5 |
| Ethylene glycol/methanol (v:v=95:5) | 0.5 |
| Isopropyl acetate | 0.3 |
| Methyl isobutyl ketone | 0.3 |
| Toluene | 0.3 |

X-ray powder diffraction analysis showed that the crystal forms obtained in Examples 11 to 16 were all crystal form I.

### Examples 17 to 21: Preparation of Crystal Form I (Cooling Method)

Approximately 50 mg of crystal form I was weighed, and dissolved in an appropriate volume of the good solvent shown in Table 4 at 50°C. The resulting solution was filtered, cooled to room temperature until a solid precipitated, and then placed in a 4°C refrigerator. When sufficient solid had precipitated, the mixture was filtered to collect the solid, which was then dried under reduced pressure (40°C).

**Table 4**

| **Solvent** | **Volume (mL)** |
|---|---|
| Ethanol | 2.0 |
| Methyl isobutyl ketone | 1.8 |
| Isopropyl acetate | 2.5 |
| Methyltetrahydrofuran | 0.8 |
| 1,4-Dioxane | 0.7 |

X-ray powder diffraction analysis showed that the crystal forms obtained in Examples 17 to 21 were all crystal form I.

### Example 22: Trituration Study of Crystal Form I

Approximately 50 mg of crystal form I was manually triturated in a mortar using the condition shown in Table 5, and XRPD was carried out to investigate the crystal form of the triturated solid.

**Table 5**

| **No.** | **Time** | **Solvent** |
|---|---|---|
| 1 | 1 min | // |
| 2 | 3 min | // |
| 3 | 1 min | Water |
| 4 | 3 min | Water |
| 5 | 1 min | Ethanol |
| 6 | 3 min | Ethanol |

The results showed that the crystal forms obtained from the above trituration study were all crystal form I. Crystal form I has good trituration stability.

### Example 23: Solubility Study of Crystal Form I

50 µL or 100 µL of the solvent shown in Table 6 was gradually added to 2 mg of crystal form I, and the resulting mixture was manually shaken, vortexed, or sonicated to dissolve. Addition of solvent was stopped if the concentration was below 1 mg/mL. The solubility was calculated based on the sample amount and the total amount of solvent added. The results are shown in Table 6.

**Table 6**

| No. | Solvent | Solubility mg/mL |
|---|---|---|
| 1 | Methanol | >19.2 |
| 2 | Acetonitrile | 14.2~28.4 |
| 3 | Acetone | >34.4 |
| 4 | Ethyl acetate | 14.9~29.8 |
| 5 | Methanol-water 1:1 | <0.895 |
| 6 | Methanol-water 9:1 | >33.4 |
| 7 | Acetonitrile-water 9:1 | >32.4 |
| 8 | pH 1.2 hydrochloric acid solution | 8.45~16.9 |
| 9 | pH 7.4 phosphate buffer (37°C for 24 hours) | 0.31 * |

| | | |
|---|---|---|
| Note: * indicates the solubility of crystal form I in pH 7.4 phosphate buffer at 37°C for 24 hours. | | |

The results showed that crystal form I had good solubility in organic solvent; moderate solubility in low pH solution (pH 1.2), ranging from 8.45 to 16.9 mg/mL; and poor solubility in high pH buffer solution (pH 7.4 phosphate buffer), with a solubility of 0.31 mg/mL at 37°C for 24 hours.

### Example 24 : Stability Study of Crystal Form I

### (1) Stability to forced degradation

3 mg of crystal form I was weighed to formulate a stock solution of 1 mg/mL with 3 mL of 90% acetonitrile. Stability subjected to oxidative degradation (degraded with 3% H₂O₂ at 40°C for 1.5 hours) and alkali degradation (degraded with 0.1 N NaOH at 40°C for 2 hours) was studied.

The results showed that crystal form I exhibited only slight degradation after being incubated in 0.1 NaOH at 40°C for 2 hour or in 3% H₂O₂ at 40°C for 1.5 hours, indicating good stability.

### (2) Stability subjected to temperature and humidity

1 to 3 mg of crystal form I solid powder was weighed, and placed at 60°C or 60°C / 75% RH for 30 days. Samples were collected for LC study and analysis.

The results showed that the content of the main component of crystal form I changed by less than 0.2% at 60°C or 60°C / 75% RH, and remained relatively stable for 30 days.

In addition, an appropriate amount of crystal form I solid powder was weighed, and placed under stability conditions (40°C / 75% RH, 60°C / 75% RH) for 30 days. Samples were collected for XRPD.

The results showed that crystal form I did not change its crystal form after being placed at 40°C / 75% RH or 60°C / 75% RH for 30 days.

### (3) Stability subjected to light

An appropriate amount of crystal form I solid powder was weighed, and placed in a light-stabilized chamber to irradiate under ICH light intensity conditions (at least greater than 1.2*10^6 Lux*h, illuminance of 4500±500 Lux, and intensity of 200 w*hr/m²). Samples were collected for LC study and analysis.

The results showed that the content of the main component of crystal form I changed by less than 0.2%, indicating that it was photostable.

### Example 25: Pharmacokinetic Study of Intragastric Administration of Suspension

Crystal form I was used in pharmacokinetic studies in rats. Twelve male SD rats were weighed before administration, and housed using standard feeding methods. Animals were housed in standard cages with free access to food and water during the study. Rats were intragastrically administered the suspension of crystal form I. 40 µL of blood was collected at each sampling point, and placed in an EDTA-containing anticoagulant tube. After centrifugation, 20 µL of plasma was collected. All samples were immediately frozen in a -80°C refrigerator after collection.

The results showed that the intragastric administration of the suspension of crystal form I resulted in high plasma concentration, high exposure, and high bioavailability in SD rats, demonstrating significant pharmacokinetic advantages.

## Claims

1. Crystal form I of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine, **characterized in that** the X-ray powder diffraction pattern thereof comprises characteristic peaks at diffraction angles (2θ) of 6.378±0.2°, 9.521±0.2°, 15.721±0.2°, 16.379±0.2°, 16.981±0.2°, 17.560±0.2°, 19.080±0.2° and 22.200±0.2°.

2. The crystal form I according to claim 1, **characterized in that** the X-ray powder diffraction pattern thereof further comprises at least one characteristic peak at diffraction angles (2θ) of 11.938±0.2°, 23.761±0.2°, 25.101±0.2° and 28.700±0.2°.

3. The crystal form I according to claim 1 or 2, **characterized in that** the X-ray powder diffraction pattern thereof comprises characteristic peaks at diffraction angles (2θ) of 6.378±0.2°, 9.521±0.2°, 11.938±0.2°, 15.721±0.2°, 16.379±0.2°, 16.981±0.2°, 17.560±0.2°, 19.080±0.2°, 22.200±0.2°, 23.761±0.2°, 25.101±0.2° and 28.700±0.2°.

4. The crystal form I according to any one of claims 1 to 3, **characterized in that** the X-ray powder diffraction pattern thereof further comprises at least one characteristic peak at diffraction angles (2θ) of 10.239+0.2°, 12.400+0.2°, 14.882+0.2°, 20.420+0.2° and 25.980+0.2°.

5. The crystal form I according to any one of claims 1 to 4, **characterized in that** the X-ray powder diffraction pattern thereof comprises characteristic peaks at diffraction angles (2θ) of 6.378+0.2°, 9.521+0.2°, 10.239+0.2°, 11.938+0.2°, 12.400+0.2°, 14.882±0.2°, 15.721±0.2°, 16.379±0.2°, 16.981±0.2°, 17.560±0.2°, 19.080±0.2°, 20.420±0.2°, 22.200±0.2°, 23.761±0.2°, 25.101±0.2°, 25.980±0.2° and 28.700±0.2°.

6. The crystal form I according to any one of claims 1 to 5, **characterized in that** the DSC spectrum thereof shows one endothermic peak at 219.25±2°C.

7. The crystal form I according to any one of claims 1 to 6, **characterized in that** the TGA spectrum thereof shows a weight loss of 0.10% from 20°C to 120°C, and a weight loss of 0.37% from 120°C to 250°C.

8. A method for preparing the crystal form I according to any one of claims 1 to 7, comprising the following steps of:
mixing 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine and an organic solvent, and collecting a solid after solid-liquid separation of the resulting suspension;
the organic solvent is one or more selected from the group consisting of petroleum ether, C₄-C₆ ether and C₂-C₆ nitrile.

9. The method according to claim 8, comprising the following steps of:
triturating 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine in an organic solvent at 0 to 30°C for 10 minutes to 2 hours, and preferably at 25°C for 30 minutes, collecting a solid after solid-liquid separation of the resulting suspension, and optionally drying the solid;
the organic solvent is one or more selected from the group consisting of petroleum ether, methyl *tert-*butyl ether, tetrahydrofuran and acetonitrile, and preferably a mixture of petroleum ether and methyl *tert-*butyl ether;
advantageously, the ratio (w/v) of 4'-cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine to the organic solvent is 1:5 to 1:1, and preferably 1:3.

10. A pharmaceutical composition, comprising the crystal form I according to any one of claims 1 to 7 and one or more pharmaceutically acceptable carrier(s).

11. A use of the crystal form I according to any one of claims 1 to 7 or the pharmaceutical composition according to claim 10 in the preparation of a medicament for treating or preventing diseases or conditions associated with inhibition of ubiquitin-specific protease 1 (USP1).

12. A use of the crystal form I according to any one of claims 1 to 7 or the pharmaceutical composition according to claim 10 in the preparation of a medicament for treating or preventing cancer,
in particular, the cancer is selected from the group consisting of lung cancer, non-small cell lung cancer (NSCLC), colon cancer, bladder cancer, osteosarcoma, ovarian cancer, skin cancer, and breast cancer.
